# EUROPEAN PATENT APPLICATION

(11) **EP 1 752 165 A1**
(43) Date of publication of application: **14.02.2007**
(21) Application number: 05721292.0
(22) Date of filing: 23.03.2005
(51) Int. Cl.: A61L 2/06, A23L 3/18, A23L 3/24, F26B 17/14

(54) **STERILIZER**

(30) Priority: 31.03.2004 JP 2004106497; 31.03.2004 JP 2004106498
(71) Applicant: Tsukasa Industry Co., Ltd., Handa-shi Aichi 475-8550 (JP)
(72) Inventor: KATO, Fumio, c/o TSUKASA INDUSTRY CO., LTD., Handa-shi, Aichi 475-8550 (JP)
(74) Representative: Mills, Julia
(86) International application number: PCT/JP2005/005192
(87) International publication number: WO 2005/097212

(57) **Abstract**

A sterilizer, having a hopper (2) allowing powders and grains to be filled therein; a first rotary valve (3) disposed under the hopper; and a second rotary valve (4) formed under the first rotary valve, wherein an airtight space (5), allowing therethrough travel of the powders and grains, is formed between the first rotary valve (3) and the second rotary valve (4), the airtight space (5) being communicated with a saturated steam injection pipe (23) injecting a saturated steam into the airtight space (5).

## Description

This application is based on Japanese patent application Nos. 2004-106497 and 2004-106498 the content of which is incorporated hereinto by reference.

### DISCLOSURE OF THE INVENTION

### Field of the Invention

The present invention relates to a sterilizer used for sterilizing powders such as source materials for foods, medicines, cosmetics, fodders, fertilizers and so forth, and so-called broken materials such as tealeaves, dried vegetables and so forth (referred to as "powders and grains", hereinafter).

### Related Art

Not all germs adhered to powders and grains are always hazardous, but suggest possibility of pollution by pathogens or food poisoning bacteria, so that sterilization is indispensable in particular for the purpose of ensuring safety of powders and grains consumed for foods. Chemical sterilization using chemicals such as ethylene oxide, propylene oxide and the like is one of ever-known sterilization methods, categorized into non-heating sterilization. These chemicals show sterilizing effects over a wide range of microorganisms, but need a long time for sterilization despite every effort of optimization in sterilization conditions, and only a few of them have been put into practical use because of fear of residue of chemicals, carcinogenesis, and problems in quality degradation. There is also a known method of ozone sterilization, showing only limited effects on species other than pathogens and Gram-negative bacteria such as *Escherichia coli*, and raising problems in considerable quality degradation due to oxidation of powders and grains. Other known non-heating sterilization methods include radiation sterilization and ultra-violet sterilization, wherein the former is not approved for food sterilization except germination prevention for potato tubers, and the latter shows only a weak sterilization power, and permeability into powders and grains is not expectable.
Considering the above, heating sterilization, in particular wet heating sterilization has widely been adopted by virtue of its wide applicability and shorter time for sterilization.

As a sterilizer adopting such wet heating sterilization, there has conventionally been proposed a sterilizer in which objects to be sterilized are placed on a conveyer belt, and exposed to steam injected against them (see Japanese Laid-Open Patent Publication No. H10-56987).

The above-described conventional sterilizer is, however, disadvantageous not only in that it occupies a large space in the horizontal direction because the objects to be sterilized are placed on a conveyer belt, but also in that it is incapable of applying a high pressure in a stable manner, and thereby incapable of injecting steam at high temperatures, and incapable of uniformly sterilizing the objects to be sterilized.

The present invention was proposed in order to solve the above-described problems in the conventional sterilizer, and is aimed at providing a novel sterilizer which can be installed in a small area, and is capable of stably applying pressure for uniform sterilization.

### SUMMARY OF THE INVENTION

Aiming at achieving the above-described objects, a first aspect of the present invention relates to a sterilizer which includes:
a hopper in which powders and grains are filled;
a first rotary valve disposed under the hopper; and
a second rotary valve formed under the first rotary valve,
wherein an airtight space, allowing therethrough travel of the powders and grains, is formed between the first rotary valve and the second rotary valve, the airtight space being communicated with a saturated steam injection pipe injecting a saturated steam into the airtight space.

The powders and grains described herein include powders such as source materials for foods, medicines, cosmetics, fodders, fertilizers and so forth, and so-called broken materials such as tealeaves, dried vegetables and so forth. In the first aspect of the present invention, it is necessary to dispose the first rotary valve and the second rotary valve in a vertical positional relation. It is also necessary for both of the first rotary valve and the second rotary valve to have air-tightness, and should not allow any gas to pass therethrough upward from the lower side of each rotary valve, and downward from the upper side of each rotary valve. The airtight space formed between the first rotary valve and the second rotary valve is a portion allowing the powders and grains discharged from the first rotary valve to pass therethrough, and the capacity is therefore not specifically limited. The saturated steam injected from the saturated steam injection pipe into the airtight space is adjusted typically to a temperature of 133°C or above (133°C to 180°C or around) under a pressure of 0.2 MPa. The saturated steam thus conditioned has characteristics of both of dry heat and wet heat, and gives a sterilization effect while successfully preventing the powders and grains from wetting.

A second aspect of the present invention relates to the sterilizer according to the first aspect, wherein the first and second rotary valves are configured as being adjustable in the rotating speeds, and configured as rotating in directions reverse to each other.

A third aspect of the present invention relates to the sterilizer according to the first or second aspect, further having a drier which includes:
an inclined plate group in which plates on one side inclined in one direction, and plates on the other side inclined in the other direction, receiving the powders and grains dropped from the plates on one side, are disposed as being alternately staggered, with a large number of fine perforations formed therein; and
a heating air supplier supplying upward a heating air from the lower side of the inclined plate group.

In the third aspect of the present invention, the powders and grains discharged from the second rotary valve move, while being fluidized by the heating air injected upward through the fine perforations formed in the individual inclined plates (inclined plate group), sequentially from the topmost inclined plate on one side to the plate on the other side, and from this inclined plate on the other side to the inclined plate disposed under the foregoing inclined plate on one side, and so on.

A fourth aspect of the present invention relates to the sterilizer according to the third aspect, wherein the inclined plate group is configured as being adjustable in the angle of inclination of each plate.

A fifth aspect of the present invention relates to the sterilizer according to the third or fourth aspect, further having a cooler disposed on the lateral of the inclined plate group,
the cooler including:
an inclined plate group in which plates on one side inclined in one direction, and plates on the other side inclined in the other direction, receiving the powders and grains dropped from the plates on one side, are disposed as being alternately staggered, with a large number of fine perforations formed therein; and
a cooling air supplier supplying upward a cooling air from the lower side of the inclined plate group.

In the first aspect of the present invention, the airtight space allowing therethrough travel of the powders and grains is formed between the first rotary valve and the second rotary valve, and the airtight space is communicated with the saturated steam injection pipe injecting a saturated steam into the airtight space, so that the pressure in the airtight space can stably be adjusted, thereby ensuring an excellent sterilizing effect and making it possible to uniformly sterilize a large amount of powders and grains.

In the second aspect of the present invention, the first and second rotary valves are configured as being adjustable in the rotating speeds, so that the rate of sterilization can arbitrarily be set. That is, the retention time in the airtight space can be adjusted depending on the pressure and temperature in the airtight space, or species, grain size and so forth of the powders and grains to be sterilized, so that the sterilizer can be made more versatile and more convenient to use. According to the second aspect of the present invention, the first and second rotary valves are configured as rotating in directions reverse to each other, raising an effect of successfully avoiding an event that the powders and grains stay long in the airtight space and would not move into the second rotary valve.

In the third aspect of the present invention, the drier is disposed under the second rotary valve, so that it is successful in avoiding an event that moisture adheres to the surface of the powders and grains after being sterilized by the saturated steam, makes the powders and grains aggregate, and adversely affects transportation after the sterilization. In particular, the drier composing the third aspect of the present invention includes an inclined plate group in which plates on one side inclined in one direction, and plates on the other side inclined in the other direction, receiving the powders and grains dropped from the plates on one side, are disposed as being alternately staggered, with a large number of fine perforations formed therein; and a heating air supplier supplying upward a heating air from the lower side of the inclined plate group, so that the powders and grains can drop by their own weight from the inclined plate group, and can effectively be dissociated even when they were discharged from the second rotary valve in a form of aggregate due to moisture adhered on the surface thereof. Furthermore, the sterilizer is configured so that the individual inclined plates have fine perforations formed therein, so as to allow the heating air to be injected upwardly therethrough, so that heating efficiency is further enhanced, the powders and grains discharged from the second rotary valve are prevented from aggregating, and the powders and grains can successfully be dissociated even when they were once aggregated.

In the fourth aspect of the present invention, the inclined plate group is configured as being adjustable in the angle of inclination of each of the plates, so that it is made possible to adjust the drying time depending on species, grain size and so forth of the powders and grains to be sterilized.

In the fifth aspect of the present invention, the sterilizer additionally has a cooler disposed on the lateral of the inclined plate group, wherein the cooler includes an inclined plate group in which plates on one side inclined in one direction, and plates on the other side inclined in the other direction, receiving the powders and grains dropped from the plates on one side, are disposed as being alternately staggered, with a large number of fine perforations formed therein; and a cooling air supplier supplying upward a cooling air from the lower side of the inclined plate group, so that the powders and grains are successfully prevented from aggregating during cooling.

### BRIEF DESCRIPTION OF THE DRAWINGS

The above and other objects, advantages and features of the present invention will be more apparent from the following description taken in conjunction with the accompanying drawings, in which:
FIG. 1 is a front view of a sterilizer according to a first embodiment;
FIG. 2 is a horizontal sectional view of the sterilizer according to the first embodiment;
FIG. 3 is a vertical sectional view of the sterilizer shown in FIG. 1;
FIG. 4 is a vertical sectional view of the sterilizer according to a second embodiment;
FIG. 5 is a right side elevation of the sterilizer according to the second embodiment; and
FIG. 6 is a plan view of the sterilizer according to the second embodiment.

A list of reference numerals is shown below:
- 1: sterilizer;
- 2: hopper;
- 3: first rotary valve;
- 4: second rotary valve;
- 5: airtight space;
- 31: sterilizer;
- 32: first hopper;
- 33: second hopper;
- 38: first rotary valve;
- 39: second rotary valve;
- 40: airtight space;
- 53: drying chamber;
- 56: heating air supply duct;
- 57: heater;
- 58: intermediate duct;
- 59: first fan;
- 63-67: first to fifth inclined plates;
- 71: cooling duct;
- 75-79: sixth to tenth inclined plates;
- 82: cooler; and
- 83: second fan.

### DETAILED DESCRIPTION OF THE PREFERRED EMBODIMENTS

The invention will be now described herein with reference to illustrative embodiments. Those skilled in the art will recognize that many alternative embodiments can be accomplished using the teachings of the present invention and that the invention is not limited to the embodiments illustrated for explanatory purposes.

Paragraphs below will detail best modes for carrying out the present invention, referring to the attached drawings. First, a sterilizer according to a first embodiment will be explained.

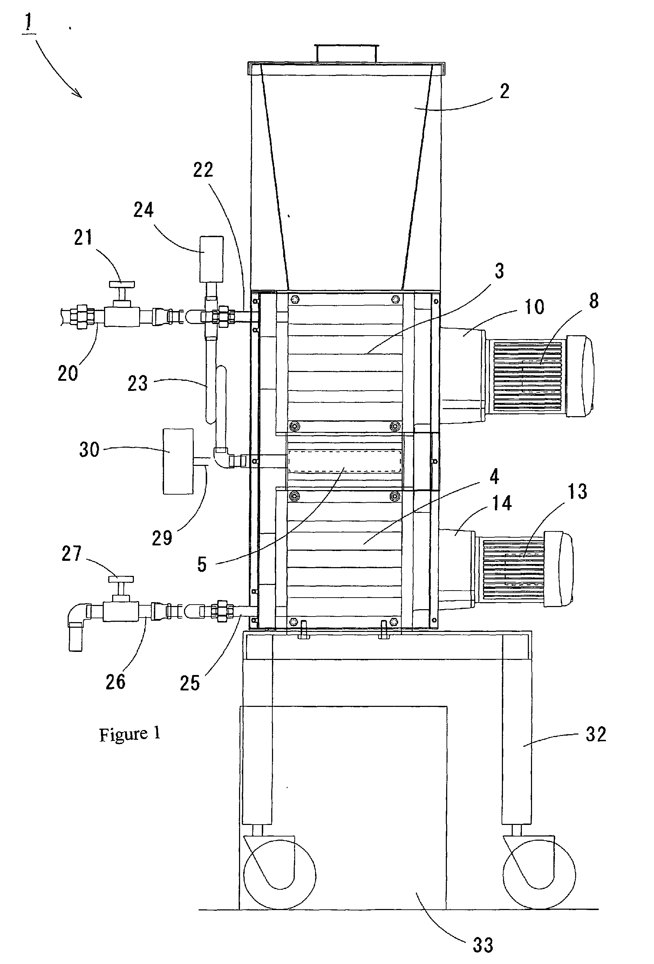

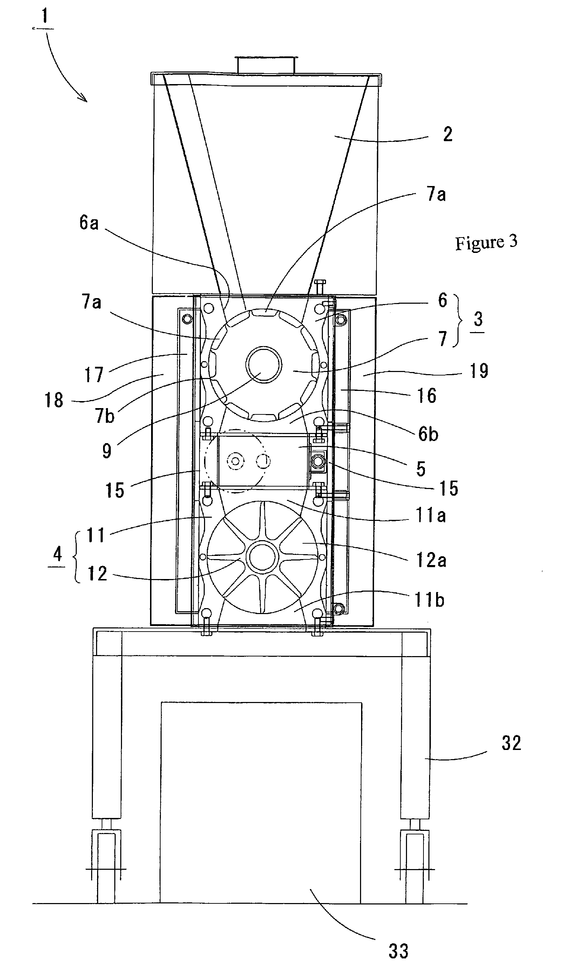

The sterilizer 1 has, as shown in FIG. 1, a hopper 2 in which powders and grains are filled, a first rotary valve 3 disposed under the hopper 2; and a second rotary valve 4 formed under the first rotary valve 3, and an airtight space 5 formed between the first rotary valve 3 and the second rotary valve 4. As shown in FIG. 1, the hopper 2 is molded in a square plain geometry which is gradually narrowed downward. The first rotary valve 3 has, as shown in FIG. 3, an enclosure 6, a main valve unit 7 housed in the enclosure 6, and a first drive motor 8 rotating the main valve unit 7. The enclosure 6 has a housing space (reference numeral not given), housing the main valve unit 7 in a rotatable manner, formed therein. The main valve unit 7 has a large number of recesses 7a formed on the outer circumference thereof, and a partition 7b between every adjacent recesses 7a. The outside dimension of the main valve unit 7 and the inside dimension of the housing space are designed as being almost equal, so that gas cannot leak between the outer circumferential surface of the main valve unit 7 and the inner circumferential surface of the housing space. The enclosure 6 has a conical intake opening 6a formed on the top surface thereof, and has a discharge opening 6b formed on the bottom surface thereof, keeping a line symmetry with the intake opening 6a. The main valve unit 7 has a rotation axis 9 fixed to the center thereof in the horizontal direction. The rotation axis 9 is a component composing a decelerator 10 shown in FIG. 1. More specifically, the first drive motor 8 is coupled via the decelerator 10 to main valve unit 7. The powders and grains passed through the intake opening 6a and housed in the recesses 7a therefore rotate with driving of the first drive motor 8, pass through the discharge opening 6b, and move by their own weight through the airtight space 5 described later.

The second rotary valve 4 is configured as being basically identical to the first rotary valve 3, and is composed of an enclosure 11, a main valve unit 12 housed in the enclosure 11 in a rotatable manner, and having a large number of recesses 12a formed on the outer circumference thereof, a second drive motor 13 rotating the main valve unit 12, and a decelerator 14. In the second rotary valve 4, the individual recesses 12b formed on the main valve unit 12 are deeper towards the center of the main valve unit 12 than those on the first rotary valve 3. It is also to be noted that twelve recesses 12a are formed on the main valve unit 7 composing the first rotary valve 3, whereas eight recesses 12a are formed in the second rotary valve 4, wherein both main valve units 7, 12 are configured as rotating in directions reverse to each other. The enclosure 11 has an intake opening 11a formed on the top surface thereof, allowing the powders and grains after traveled across the airtight space 5 to flow therethrough, and has a discharge opening 11b formed on the bottom surface thereof, allowing therethrough discharge of the powders and grains. The rotating speeds of the first rotary valve 3 and the second rotary valve 4 are independently adjustable.

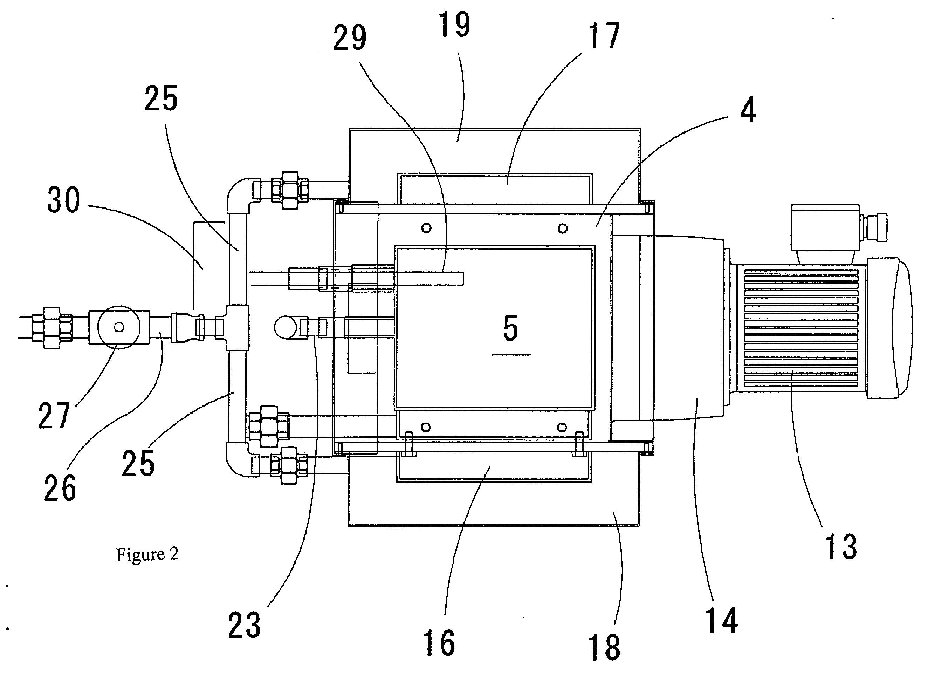

The first rotary valve 3 and the second rotary valve 4 are spaced from each other, four sides are individually fixed onto fixation plates 15, and the airtight space 5 is formed inside. In other words, the discharge opening 6b formed to the first rotary valve 3 is located on the upper side of the airtight space 5, and the intake opening 11a formed to the second rotary valve 4 is located on the lower side. In the sterilizer 1 according to this embodiment, there are fixed a front-side heating component 16 and a back-side heating component 17 extending so as to lay the longitudinal direction thereof over the range from the top end side of the first rotary valve 3 to the bottom end side of the second rotary valve 4. The front-side heating component 16 is fixed to the front of the sterilizer 1 as shown in FIG. 2, and has a steam flow space (reference numeral not given) formed therein. The back-side heating component 17 is fixed to back side of the sterilizer 1, and again has a steam flow space (reference numeral not given) formed therein. The front side and both flanks of the front-side heating component 16 are covered with a heat insulator 18, and the front side and both flanks of the back-side heating component 17 are again covered with a heat insulator 19.

The sterilizer 1 has a supply pipe 20 supplying saturated steam into the sterilizer 1, as shown in FIG. 1. The supply pipe 20 is connected on the upstream side thereof to a boiler not shown, has a first manual valve 21 on the midway, and connected on the downstream side of the first manual valve 21 to a first branch pipe 22 communicated to the inner portions of the front-side heating component 16 and the back-side heating component 17, and a second branch pipe 23 supplying the saturated steam into the airtight space 5. The second branch pipe 23 corresponds to the saturated steam injection pipe composing the present invention. A pressure gauge 24 is provided on the upstream side of the second branch pipe 23, configured so as to allow the operator to monitor the pressure of the saturated steam supplied into the airtight space 5. On the midway on the bottom end side of each of the front-side heating component 16 and the back-side heating component 17, a discharge-side branch pipe 25 discharging the saturated steam flown into the front-side heating component 16 and the back-side heating component 17 is provided, and both discharge-side branch pipes 25 are connected to a discharge pipe 26. A second manual valve 27 is provided on the midway of the discharge pipe 26.

In the airtight space 5 connected with the second branch pipe 23, one end of a discharge pipe 29 discharging the saturated steam supplied into the airtight space 5 is inserted, and a sound insulator 30 is connected on the other end of the discharge pipe 29. The sterilizer 1 is installed and fixed on a movable carriage 32 as shown in FIG. 1 and FIG. 3, and a housing box 33 housing the powders and grains sterilized by the sterilizer 1 is disposed inside the movable carriage 32.

In thus-configured sterilizer 1 according to the first embodiment, the powders and grains thrown into the hopper 2 are housed in the recesses 7a formed on the main valve unit 7 composing the first rotary valve 3, and are supplied by small portions into the airtight space 5 as the rotation axis 9 rotates. The powders and grains supplied into the airtight space 5 are sterilized by the saturated steam supplied through the second branch pipe 23 at a temperature of approximately 133°C or above, housed in the recesses 12a formed on the main vale unit 12 composing the second rotary valve 4 and rotating in the direction reverse to the main valve unit 7, allowed to pass through the enclosure 11, discharged through the discharge opening 11b, and are housed in the housing box 33.

Because the powders and grains are sterilized on the way from the first rotary valve 3 disposed on the upper side towards the second rotary valve 4 disposed under the first rotary valve 3, the sterilizer 1 can be installed in a small area without needing a wide space. In particular in this sterilizer 1, not only the airtight space 5 is configured as being air-tight, but also the first and second rotary valves 3, 4 can supply and discharge the powders and grains to or from the airtight space 5 while keeping air-tightness, so that the sterilizer 1 can keep a stable air-tightness, without causing variation in the sterilization. In the sterilizer 1, not only the powders and grains are sterilized under heating in the airtight space 5 formed between the first rotary valve 3 and the second rotary valve 4, but also the entire portions of the first and second rotary valves 3, 4 are heated by the saturated steam supplied into the front-side heating component 16 and the back-side heating component 17, so that heating loss can be reduced in an extremely effective manner, and thereby a satisfactory level of energy saving can be expected.

The sterilizer 1 is configured as being arbitrarily adjustable in the rotation speeds of the first rotary valve 3 and the second rotary valve 4, so that the retention time of the powders and grains in the airtight space 5 can appropriately be adjusted depending on species, grain size and so forth of the powders and grains. Because the directions of rotation of the first rotary valve 3 and the second rotary valve 4 are set reverse to each other, the powders and grains are successfully prevented from staying long in the airtight space 5 without being supplied to the second rotary valve 4.

Paragraphs below will detail a sterilizer 31 according to a second embodiment of the present invention.

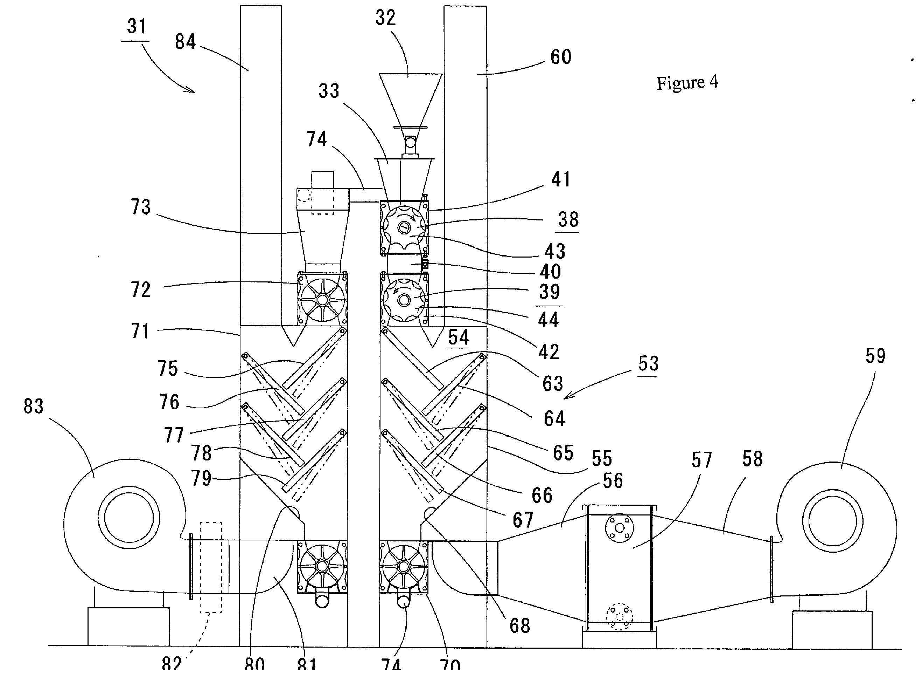

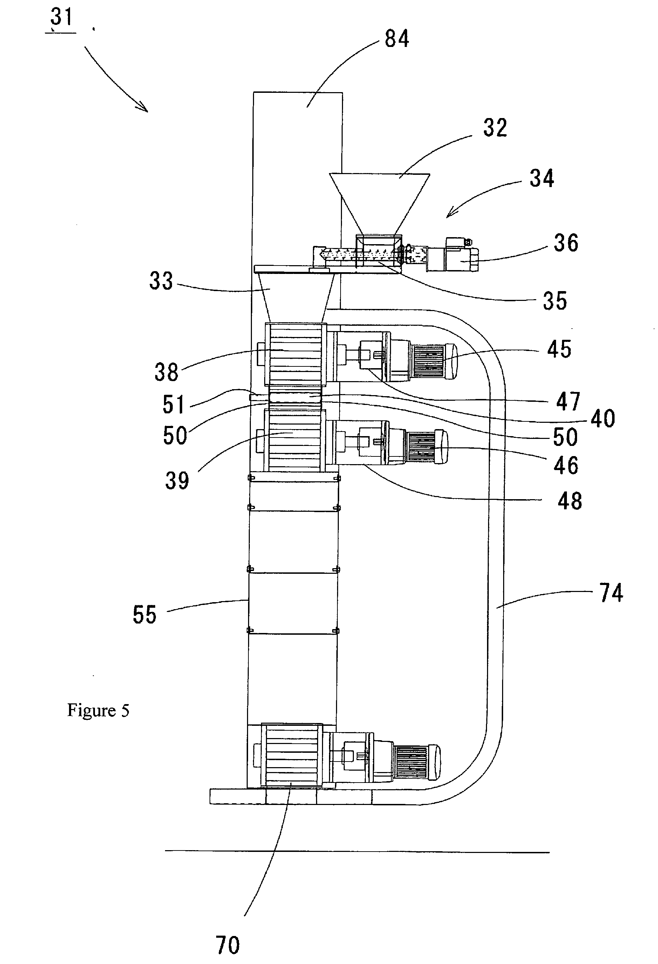

As shown in FIG. 4, the sterilizer 31 has a first hopper 32 in which the powders and grains are filled, a second hopper disposed in the vicinity of the first hoper 32, and a feeder 34 (see FIG. 5) feeding the powders and grains thrown into the first hopper 32 into the second hopper 33. The feeder 34 is composed of, as shown in FIG. 5, a feeding pipe (reference numeral not given) disposed under the first hopper 32, and having a screw 35 incorporated therein, and a first drive motor 36 rotating the screw 35, with the end of the feeding pipe located above the second hopper 33. A first rotary valve 38 is disposed under the second hopper 33, as shown in FIG. 4 and FIG. 5, a second rotary valve 39 is disposed under the first rotary valve 38, and an airtight space 40 composing the present invention is formed between the first rotary valve 38 and the second rotary valve 39. The first rotary valve 38 and the second rotary valve 39 are configured similarly to each other, respectively having enclosures 41, 42, main valve units 43, 44 housed in the enclosures 41, 42 in a rotatable manner, with a large number of recesses (reference numeral not given) formed on the outer circumferences thereof, second and third drive motors 45, 46 (see FIG. 5) rotating the main valve units 43, 44, and decelerators 47, 48 disposed respectively between the second and third drive motors 45, 46 and the main valve units 43, 44. The airtight space 40 is surrounded on four sides thereof by fixation plates 40 while keeping air-tightness, and is connected to a saturated steam supply pipe 51 as shown in FIG. 5. The saturated steam supply pipe 51 is connected on the upstream side thereof to a boiler not shown, with the other end side communicated to the airtight space 40.

The second rotary valve 39 is placed on a drying chamber 53 as shown in FIG. 4. The drying chamber 53 has a drying duct 55 having, as being formed therein, a drying space 54 communicated to a discharge opening (reference numeral not given) formed on the bottom surface of the second rotary valve 39, a heating air supply duct 56 communicated to the drying duct 55, a heater 57 connected to the heating air supply duct 56, and a first fan 59 connected through an intermediate duct 58 to the heater 57. The heating air supply duct 56, the heater 57, the intermediate duct 58 and the first fan 59 correspond to the heating air supplier composing the present invention. The drying duct 55 extends so as to align the longitudinal direction thereof with the perpendicular direction. On the lateral of the second rotary valve 39, there is fixed the lower end of a first exhaust duct 60. The upper end of the first exhaust duct 60 is positioned higher than the upper end of the first hopper 32. In the drying duct 55, there are disposed a first to fifth inclined plates 63...67. The first to fifth inclined plates 63...67 correspond to the inclined plate group composing the present invention. The first inclined plate 63 is disposed so as to locate the mid portion thereof below the second rotary valve 39, as being inclined downward towards the distal end. The second inclined plate 64 is provided so as to receive the powders and grains dropped from the first inclined plate 63, and inclined downward from the proximal end towards the distal end thereof. The third inclined plate 65 is disposed under the first inclined plate 63 so as to receive the powders and grains dropped from the second inclined plate 64, and inclined downward from the proximal end towards the distal end thereof. The fourth inclined plate 66 is disposed under the second inclined plate 64 so as to receive the powders and grains dropped from the third inclined plate 65, and inclined downward from the proximal end towards the distal end thereof. The fifth inclined plate 67 is disposed under the third inclined plate 65 so as to receive the powders and grains dropped from the fourth inclined plate 66, and inclined downward from the proximal end towards the distal end thereof. The first to fifth inclined plates 63...67 have a large number of unillustrated fine perforations formed therein, allowing the heating air to pass upward therethrough. On the lateral side of the fifth inclined plate 67 and under the fourth inclined plate 66, there is provided an inclined plate portion 68. The inclined plate portion 68 has a large number of unillustrated fine perforations formed therein. The first to fifth inclined plates 63...67 are configured as being independently adjustable in the angle of inclination, as indicated by two-dot chain lines in FIG. 4. The first inclined plate 63, the third inclined plate 65 and the fifth inclined plate 67 correspond to the inclined plates on one side composing the present invention, whereas the second inclined plate 64 and the fourth inclined plate 66 correspond to the inclined plates on the other side composing the present invention.

The drying duct 55 and the heating air supply duct 56 are connected at a position below the inclined plate portion 68 formed in the drying duct 55. Below the fifth inclined plate 67, there is disposed a third rotary valve 70. In this configuration, the powders and grains discharged from the second rotary valve 39 gradually drop as being sequentially received by the first inclined plate 63, the second inclined plate 64, the third inclined plate 65, the fourth inclined plate 66 and the fifth inclined plate 67 in this order, and finally reach the third rotary valve 70 via the inclined plate portion 68. During this mode of travel, the powders and grains are dried under heating by the heating air moving upward through a large number of fine perforations formed in the second to fifth inclined plates 64...67.

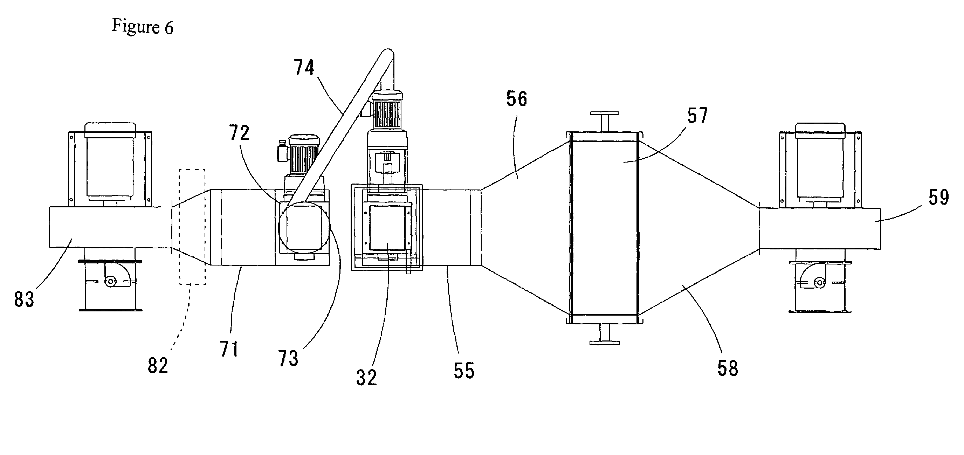

On the lateral of the drying duct 55, there is disposed a cooling duct 71 having the same height with the drying duct 55, and a fourth rotary valve 72 is placed and fixed on the cooling duct 71. More specifically, a cooler (reference numeral not given) is disposed in adjacent to the drying chamber 53. A third hopper 73 is disposed on the fourth rotary valve 72. The third hopper 73 is connected, as shown in FIG. 6, via a relay pipe 74 to the third rotary valve 70. The cooling duct 71 has, similarly to the drying duct 55, the sixth to tenth inclined plates 75...79 disposed therein. The sixth to tenth inclined plates 75...79 have a large number of fine perforations formed therein, similarly to as in the first to fifth inclined plates 63...67, and are configured as being independently adjustable in the angle of inclination. On the diagonally lower side of the tenth inclined plate 78, there is disposed an inclined plate portion 80 having a large number of unillustrated fine perforations formed therein. To the cooling duct 71, there is fixed the proximal end of a second exhaust duct 84 through which the cooling air supplied into the cooling duct is discharged. The second exhaust duct 84 is disposed on the lateral of the fourth rotary valve 72, and has the same height with the first exhaust duct 60.

On the lower end side of the cooling duct 71, a cooling air supply duct 81 is connected, and a second fan 83 integrated with a cooler (chiller) 82 is connected to the proximal end of the cooling air supply duct 81. By operation of the second fan 83 and the cooler 82, the chilled cooling air is supplied through the cooling air supply duct 81 to the lower side of the inclined plate portion 80 composing the cooling duct 71, allowed to flow through the large number of fine perforations formed in the inclined plate portion 80 into the cooling duct 71, and further allowed to flow through the fine perforations respectively formed in the sixth to tenth inclined plates 75...79, and discharged through the second exhaust duct 84 into the air.

Therefore in addition to the operations and effects same as those obtained by the sterilizer 1 according to the first embodiment, the sterilizer 31 according to the second embodiment can not only effectively prevent the powders and grains sterilized by the saturated steam from aggregating due to dewing, but can also dissociate the powders and grains even if they were aggregated, by allowing them to pass through the drying duct 55. Furthermore in the sterilizer 31, the powders and grains passed through the drying duct 55 are further allowed to pass through the cooling duct 71, so that they are more effectively prevented from being conveyed to elsewhere while keeping an aggregated form. The drying duct 55 and cooling duct 71 adopt a system allowing the powders and grains to move downward so as to drop them on the plurality of inclined plates (reference numerals not given), and the individual inclined plates are configured as having a large number of fine perforations allowing therethrough upward injection of the heating air and the cooling air, so that the sterilizer can effectively improve the drying and cooling efficiencies, without needing a large area for installation.
It is apparent that the present invention is not limited to the above embodiments, that may be modified and changed without departing from the scope and spirit of the invention.

## Claims

1. A sterilizer comprising:
a hopper in which powders and grains are filled;
a first rotary valve disposed under said hopper; and
a second rotary valve formed under said first rotary valve,
wherein an airtight space, allowing therethrough travel of said powders and grains, is formed between said first rotary valve and said second rotary valve, said airtight space being communicated with a saturated steam injection pipe injecting a saturated steam into said airtight space.

2. The sterilizer as claimed in Claim 1, wherein said first and second rotary valves are configured as being adjustable in the rotating speeds, and configured as rotating in directions reverse to each other.

3. The sterilizer as claimed in Claim 1 or 2, further comprising a drier which includes:
an inclined plate group in which plates on one side inclined in one direction, and plates on the other side inclined in the other direction, receiving the powders and grains dropped from said plates on one side, are disposed as being alternately staggered, with a large number of fine perforations formed therein; and
a heating air supplier supplying upward a heating air from the lower side of said inclined plate group.

4. The sterilizer as claimed in Claim 3, wherein said inclined plate group is configured as being adjustable in the angle of inclination of each of said plates.

5. The sterilizer as claimed in Claim 3 or 4 further comprising a cooler disposed on the lateral of said inclined plate group,
said cooler including:
an inclined plate group in which plates on one side inclined in one direction, and plates on the other side inclined in the other direction, receiving the powders and grains dropped from said plates on one side, are disposed as being alternately staggered, with a large number of fine perforations formed therein; and
a cooling air supplier supplying upward a cooling air from the lower side of said inclined plate group.
